**Europäisches Patentamt**

(19) **European Patent Office**

**Office européen des brevets**

(11) Publication number: **0 117 059**
**B1**

(12) **EUROPEAN PATENT SPECIFICATION**

(45) Date of publication of patent specification: **27.12.90**

(21) Application number: **84300299.9**

(22) Date of filing: **18.01.84**

(51) Int. Cl.⁵: **C 12 N 15/85, C 12 N 9/72, C 12 N 9/06**

(54) Methods for producing human tPA and expression vectors therefor.

(30) Priority: **19.01.83 US 459153**

(43) Date of publication of application:
**29.08.84 Bulletin 84/35**

(45) Publication of the grant of the patent:
**27.12.90 Bulletin 90/52**

(84) Designated Contracting States:
**AT BE CH DE FR GB IT LI LU NL SE**

(56) References cited:
**EP-A-0 037 687**
**EP-A-0 093 619**
**EP-A-0 117 060**

**PROCEEDINGS OF THE NATIONAL ACADEMY OF SCIENCES USA, vol. 77, o. 6, June 1980, pages 3567-3570; M. WIGLER et al.: "Transformation of mammalian cells with an amplifiable dominant-acting gene"**

(73) Proprietor: **GENENTECH, INC.**
**460 Point San Bruno Boulevard**
**South San Francisco California 94080 (US)**

(72) Inventor: **Levinson, Arthur David**
**3690 Ralston Avenue**
**Hillsborough California 94010 (US)**
Inventor: **Simonsen, Christian Clinton**
**1509 Parkview Avenue**
**San Jose California 95310 (US)**
Inventor: **Yelverton, Elizabeth McLeod**
**1624 Danromas Way**
**San Jose California 95129 (US)**

(74) Representative: **Armitage, Ian Michael et al**
**MEWBURN ELLIS & CO. 2/3 Cursitor Street**
**London EC4A 1BQ (GB)**

Courier Press, Leamington Spa, England.

**Description**

Background of the invention

The invention herein relates to the production of human tissue plasminogen activator (tPA) in a transformant host cell culture. More specifically, the invention relates to vectors, cells, and methods of producing tPA in conjunction with expression of the sequences for coding for dihydrofolate reductase (DHFR) protein in such cells.

The production of tPA using recombinant techniques has been disclosed in U.S. Applications Serial No. 374,860, filed 5 May 1982, Serial No. 398,003, filed 14 July 1982 and Serial No. 483,052, filed 7 April 1983, corresponding to European Patent Office Publication Number 0093619, the contents of which are herein incorporated by reference. These applications describe the construction of plasmids containing the coding sequences for tPA, and describe the activity and utility of tPA so produced.

It is also been found, as set forth in U.S. Applications Serial No. 459,152, filed 19 January 1983 and in EP 117060 A incorporated herein by reference, that a DNA sequence encoding for a DHFR protein can be utilized as a marker for transfection of a sequence coding for a desired heterologous protein in suitable host cells. The DHFR sequence can also be used as a secondary sequence permitting control of the production of the desired protein. These applications disclose such a use, both of wild type DHFR, and of a mutant DHFR which is resistant to methotrexate.

A problem frequently encountered in the production of polypeptides in a foreign host is the necessity to have some mechanism to regulate, usually to enhance, the production of the desired protein. In the case of tPA, which forms the subject matter of this invention, a secondary coding sequence comprising DHFR which is affected by an externally controlled parameter, such as methotrexate, is utilized to permit control of expression by control of the methotrexate (MTX) concentration.

Methotrexate is a drug which is normally fatal to cells capable of its uptake. However, certain cells are able to grow in the presence of controlled levels of MTX. One of the several mechanisms whereby methotrexate resistance is effected is that whereby amplification of the gene coding for the DHFR coding sequence is stimulated (Schimke, Robert T. et al, *Science*, 202: 1051 (1978); Biedler, J. I. et al, *Cancer Res.* 32: 153 (1972); Chang, S. E., et al, *Cell*, 7: 391 (1976)).

It has further been shown that amplification of the gene for DHFR may further cause amplification of associated sequences which code for other proteins. This appears to be the case when the associated protein is hepatitis B surface antigen (HBsAg) (Christman, J. et al, *Proc. Natl. Acad. Sci.*, 79: 1815 (1982)); the *E. coli* protein XGPRT (Ringold, Gordon, et al, *J. Molec. and Appl. Gen.*, 1: 165 (1981)); and an endogenous sequence from a DHFR/SV40 plasmid combination (Kaufman, R. F. et al, *J. Molec. Biol.*, 159: 601 (1982)).

Other mechanisms for conferring methotrexate resistance include diminution of the binding affinity of the DHFR protein, so that it is less susceptible to methotrexate (Flintoff, W. F. et al, *Somat. Cell Genet.*, 2: 245 (1976)) but in this instance, amplification appears to occur as well.

Thus it would appear that the genes both for wild type DHFR and for DHFR which is resistant to MTX by virtue of its own decreased binding capacity are amplified by the presence of MTX. Hence, in principle, the invention herein concerns using the impact of DHFR sequence amplification on associated protein coding sequences to provide a control mechanism which permits enhanced expression levels of tPA sequences in the presence of MTX, or by virtue of prior treatment of transformed cells with MTX.

As described in EPO Patent Application Publn. No. 0093619, tPA is a fibrinolytic substance which can be recovered from human melanoma cells (see also EPO Patent Application Publn. No. 0041766). This product has been isolated and characterized [Weiman et al, *The Lancet*, II (8250): 1018 (1981)]. Its fibrinolytic activity is analogous to that of two commercially available proteins, streptokinase and urokinase, which are indicated for the treatment of acute cardiovascular diseases such as myocardial infarct, stroke, pulmonary embolism, deep vein thrombosis, peripheral arterial occlusion and other venous thromboses. The etiological basis for these diseases is apparently either a partial or total occlusion of a blood vessel by a blood clot. Thus traditional anticoagulant therapy for example, treatment with heparin or coumarin, is not effective as it will merely prevent the formation of further clots, but not result in the dissolution of clots already formed. The fibrinolytic agents, streptokinase, urokinase, and plasminogen activator all operate similarly. They convert the inactive precursor plasminogen into plasmin which is capable of dissolving the fibrin of which these clots are composed. Plasminogen activator has a high affinity for fibrin, and thus preferentially activates plasminogen associated with the fibrin desired to be dissolved. On the other hand, streptokinase and urokinase do not; hence, much of the plasmin formed is formed in circulating blood and is neutralized before it can reach the targeted clot. Furthermore, as these compounds create circulating rather than fibrin bound plasmin, other clotting factor proteins in circulation such as fibrinogen, Factor V, and Factor VIII are also attacked by the activated protein causing a hermorrhagic potential. Furthermore, streptokinase is strongly immunogenic.

Plasminogen activator overcomes the foregoing difficulties by specifically attacking plasminogen already bound to fibrin. The present invention concerns a method of increasing and controlling the production of this valuable protein in recombinant cultures by effecting control on amplification of the sequence for DHFR protein.

As used herein, "human tissue plasminogen activator" or "human t-PA" or "t-PA" denotes human extrinsic (tissue-type) plasminogen activator, produced by microbial or cell culture systems, in bioactive forms comprising a protease portion and corresponding to those tissue plasminogen activators otherwise native to human tissue. The human tissue plasminogen activator protein produced herein has been defined by means of determined DNA gene and deductive amino acid sequencing. It will be understood that natural allelic variations exist and occur from individual to individual. These variations may be demonstrated by (an) amino acid difference(s) in the overall sequence or by deletions, substitutions, insertions, inversions or additions of (an) amino acid(s) in said sequence. In addition, the location of and degree of glycosylation will depend on the nature of the host cellular environment.

The potential exists, in the use of recombinant DNA technology, for the preparation of various human tissue plasminogen activator derivatives, variously modified by resultant single or multiple amino acid substitutions, deletions, additions or replacements, for example, by means of site directed mutagenesis of the underlying DNA. Included would be the preparation of derivatives retaining the essential kringle region and serine protease region characteristic generally of the human tissue plasminogen activator described specifically herein, but otherwise modified as described above. All such allelic variations and modifications resulting in derivatives of human tissue plasminogen activator are included as well as other related human extrinsic (tissue-type) plasminogen activators, similar physically and biologically, so long as the essential, characteristic human tissue plasminogen activator activity remains unaffected in kind.

Human tissue plasminogen activator is prepared 1) having methionine as its first amino acid (present by virtue of the ATG start signal codon insertion in front of the structural gene) or 2) where the methionine is intra- or extracellularly cleaved, having its normally first amino acid, or 3) together with either its signal polypeptide or a conjugated protein other than the conventional signal polypeptide, the signal polypeptide or conjugate being specifically cleavable in an intra- or extracellular environment or 4) by direct expression in mature form without the necessity of cleaving away any extraneous, superflous polypeptide. The latter is particularly important where a given host may not, or not efficiently, remove a signal peptide where the expression vehicle is designed to express the tissue plasminogen activator together with its signal peptide. In any event, the thus produced human t-PA, in its various forms, is recovered and purified to a level fitting it for use in the treatment of various vascular conditions or diseases.

Furthermore, t-PA has forms which include both the single chain (1-chain) protein and the 2-chain protein. The latter is proteolytically derived from the 1-chain compound. It is theorized that the 2-chain protein is associated with produced fibrin and that proteolytic conversion from 1- to 2- chain material occurs at the locus of the conversion of plasminogen to plasmin. There is therefore the possibility of the administration of the 1-chain protein for *in vivo* conversion as just described or the administration of 2-chain protein, which has also been shown to be active. The 2-chain protein can be prepared by *in vitro* proteolytic conversion after the 1-chain material is produced. A so-called "kringle" area is positioned upstream from the serine protease portion and is believed to play an important function is binding the tissue plasminogen activator hereof to a fibrin matrix; hence, the observed specific activity of the present tissue plasminogen activator toward tangible, extant thrombi. The tissue plasminogen activator hereof is produced containing the enzymatically active portion corresponding to native material and the term human tissue plasminogen activator defines products comprising such portion alone or together with additional amino acid sequences up to the full length molecule.

Human t-PA thus has a functional definition; it is capable of catalyzing the conversion of plasminogen to plasmin, binds to fibrin, and is classified as a t-PA based on immunological properties as set forth hereinabove.

Summary of the invention

In one aspect, the invention herein concerns plasmids which contain coding sequences for human tissue plasminogen activator (tPA) and a DHFR protein, and which are effective in expressing both of them. In another aspect, the invention concerns cells transformed with these vectors.

In other aspects, the invention also concerns methods for producing tPA by taking advantage of the environmentally controlled response of DHFR coding sequences co-transfected with the tPA sequence, and the tPA so produced.

The invention includes human tPA which is produced by recombinant host cells, preferably in amounts greater than 0.1 pg per cell per day, most preferably in amounts greater than 20 pg per cell per day.

The invention further includes human tPA which is produced by recombinant host cells, preferably in amounts greater than $9 \times 10^{-6}$ units per cell per day, most preferably in amounts greater than $18 \times 10^{-5}$ units per cell per day.

Brief description of the drawings

Figure 1 is a schematic diagram of the construction of the exemplified DHFR (mutant or wild type)/tPA encoding plasmids.

Figures 2 (a, b and c) illustrates the nucleotide sequence and deduced amino acid sequence of the full length human tissue plasminogen activator c-DNA.

## A. Definitions

As used herein:

Human "tissue plasminogen activator" (tPA) is a fibrinolytic protein as described in EPO Patent Application Publn. No. 0041766, incorporated herein by reference.

"DHFR protein" refers to a protein which is capable of the activity associated with dihydrofolate reductase (DHFR) and which, therefore, is required to be produced by cells which are capable of survival on medium deficient in hypoxanthine, glycine, and thymidine (-HGT medium). In general, cells lacking DHFR protein are incapable of growing on this medium, cells which contain DHFR protein are successful in doing so.

"Cells sensitive to MTX" refers to cells which are incapable of growing on media which contain the DHFR inhibitor methotrexate (MTX). Thus, "cells sensitive to MTX" are cells which, unless genetically altered or otherwise supplemented, will fail to grow under ambient and medium conditions suitable for the cell type when the MTX concentration is 0.2 µg/ml or more. Some cells, such as bacteria, fail to exhibit MTX sensitivity due to their failure to permit MTX inside their cell boundaries, even though they contain DHFR which would otherwise be sensitive to this drug. In general, cells which contain, as their DHFR protein, wild type DHFR will be sensitive to methotrexate if they are permeable or capable of uptake with respect to MTX.

"Wild type DHFR" refers to dihydrofolate reductase as is ordinarily found in the particular organism in question. Wild type DHFR is generally sensitive *in vitro* to low concentrations of methotrexate.

"DHFR protein with low binding affinity for MTX" has a functional definition. This is a DHFR protein which, when generated within cells, will permit the growth of MTX sensitive cells in a medium containing 0.2 µg/ml or more of MTX. It is recognized that such a functional definition depends on the facility with which the organism produces the "DHFR protein with low binding affinity for MTX" as well as upon the protein itself. However, as used in the context of this invention, such a balance between these two mechanisms should not be troublesome. The invention operates with respect to conferring the capability of surviving these levels of MTX, and it is not consequential whether the ability to do so is impacted by increased expression in addition to the innate nature of the DHFR produced.

"Expression vector" includes vectors which are capable of expressing DNA sequences contained therein, where such sequences are operably linked to other sequences capable of effecting their expression. It is implied, although not always explicitly stated, that these expression vectors must be replicable in the host organisms either as episomes or as an integral part of the chromosomal DNA. Clearly a lack of replicability would render them effectively inoperable. In sum, "expression vector" is given a functional definition, and any DNA sequence which is capable of effecting expression of a specified DNA code disposed therein is included in this term as it is applied to the specified sequence. In general, expression vectors of utility in recombinant DNA techniques are often in the form of "plasmids" which refer to circular double stranded DNA loops which, in their vector form are not bound to the chromosome. In the present specification, "plasmid" and "vector" are used interchangeably as the plasmid is the most commonly used form of vector. However, the invention is intended to include such other forms of expression vectors which serve equivalent functions and which become known in the art subsequently hereto.

"Recombinant host cells" refers to cells which have been transformed with vectors constructed using recombinant DNA techniques. As defined herein, tPA produced in the amounts achieved by virtue of this transformation, rather than in such lesser amounts, or, more commonly, in such less than detectable amounts, as would be produced by the untransformed host.

## B. Detailed description
### B.1 Host cell cultures and vectors

The vectors and methods disclosed herein are suitable for use in host cells over a wide range of prokaryotic and eukaryotic organisms.

In general, of course, prokaryotes are preferred for cloning of DNA sequences in constructing the vectors useful in the invention. For example, E. coli K12 strain 294 (ATCC No. 31446) is particularly useful. Other microbial strains which may be used include E. coli strains such as E. coli B, and E. coli X1776 (ATCC No. 31537). These examples are, of course, intended to be illustrative rather than limiting.

Prokaryotes may also be used for expression. The aforementioned strains, as well as E. coli W3110 (F⁻, λ⁻, prototrophic, ATTC No. 27325), bacilli such as *Bacillus subtilus*, and other enterobacteriaceae such as *Salmonella typhimurium* or *Serratia marcesans*, and various pseudomonas species may be used.

In general, plasmid vectors containing replicon and control sequences which are derived from species compatible with the host cell are used in connection with these hosts. The vector ordinarily carries a replication site, as well as marking sequences which are capable of providing phenotypic selection in transformed cells. For example, E. coli is typically transformed using pBR 322, a plasmid derived from an E. coli species (Bolivar, et al., *Gene* 2: 95 (1977)). pBR322 contains genes for ampicillin and tetracycline resistance and thus provides easy means for identifying transformed cells. The pBR322 plasmid, or other microbial plasmid must also contain, or be modified to contain, promoters which can be used by the microbial organism for expression of its own proteins. Those promoters most commonly used in recombinant DNA construction include the β-lactamase (penicillinase) and lactose promoter systems (Chang et al, *Nature*, 275: 617 (1978); Itakura, et al, *Science*, 198: 1056 (1977); (Goeddel, et al *Nature* 281:

544 (1979)) and a tryptophan (trp) promoter system (Goeddel, et al, *Nucleic Acids Res.*, 8: 4057 (1980); EPO Appl Publ No. 0036776). While these are the most commonly used, other microbial promoters have been discovered and utilized, and details concerning their nucleotide sequences have been published, enabling a skilled worker to ligate them functionally with plasmid vectors (Siebenlist, et al, *Cell* 20: 269 (1980)).

In addition to prokaryotes, eukaryotic microbes, such as yeast cultures may also be used. *Saccharomyces cerevisiae*, or common baker's yeast is the most commonly used among eukaryotic microorganisms, although a number of other strains are commonly available. For expression in *Saccharomyces*, the plasmid YRp7, for example, (Stinchcomb, et al, *Nature*, 282: 39 (1979); Kingsman et al, *Gene*, 7: 141 (1979); Tschemper, et al, *Gene*, 10: 157 (1980)) is commonly used. This plasmid already contains the *trp*l gene which provides a selection marker for a mutant strain of yeast lacking the ability to grow in tryptophan, for example ATCC No. 44076 or PEP4-1 (Jones, *Genetics*, 85: 12 (1977)). The presence of the *trp*l lesion as a characteristic of the yeast host cell genome then provides an effective environment for detecting transformation by growth in the absence of tryptophan.

Suitable promoting sequences in yeast vectors include the promoters for 3-phosphoglycerate kinase (Hitzeman, et al., *J. Biol. Chem.*, 255: 2073 (1980)) or other glycolytic enzymes (Hess, et al, *J. Adv. Enzyme Reg.*, 7: 149 (1968); Holland, et al, *Biochemistry*, 17: 4900 (1978)), such as enolase, glyceraldehyde-3-phosphate dehydrogenase, hexokinase, pyruvate decarboxylase, phosphofructokinase, glycose-6-phosphate isomerase, 3-phosphoglycerate mutase, pyruvate kinase, triosephosphate isomerase, phosphoglucose isomerase, and glucokinase. In constructing suitable expression plasmids, the termination sequences associated with these genes are also ligated into the expression vector 3' of the sequence desired to be expressed to provide polyadenylation of the mRNA and termination. Other promoters, which have the additional advantage of transcription controlled by growth conditions are the promoter regions for alcohol dehydrogenase 2, isocytochrome C, acid phosphatase, degradative enzymes associated with nitrogen metabolism, and the aforementioned glyceraldehyde-3-phosphate dehydrogenase, and enzymes responsible for maltose and galactose utilization (Holland, ibid.). Any plasmid vector containing yeast-compatible promoter, origin of replication and termination sequences is suitable.

In addition to microorganisms, cultures of cells derived from multicellular organisms may also be used as hosts. In principle, any such cell culture is workable, whether from vertebrate or invertebrate culture. However interest has been greatest in vertebrate cells, and propagation of vertebrate cells in culture (tissue culture) has become a routine procedure in recent years [*Tissue Culture*, Academic Press, Kruse and Patterson, editors (1973)]. Examples of such useful host cell lines are VERO and HeLa cells, Chinese hamster ovary (CHO) cell lines, and W138, BHK, COS-7 and MDCK cell lines. Expression vectors for such cells ordinarily include (if necessary) an origin of replication, a promoter located in front of the gene to be expressed, along with any necessary ribosome binding sites, RNA splice sites, polyadenylation site, and transcriptional terminator sequences. It will be understood that this invention, although described herein in terms of a preferred embodiment, should not be construed as limited to those sequences exemplified.

For use in mammalian cells, the control functions on the expression vectors are often provided by viral material. For example, commonly used promoters are derived from polyoma, Adenovirus 2, and most frequently Simian Virus 40 (SV40). The early and late promoters of SV40 virus are particularly useful because both are obtained easily from the virus as a fragment which also contains the SV40 viral origin of replication (Friers, et al, *Nature*, 273: 113 (1978) incorporated herein by reference. Smaller or larger SV40 fragments may also be used, provided there is included the approximately 250 bp sequence extending from the Hind III site toward the Bgl I site located in the viral origin of replication. Further, it is also possible, and often desirable, to utilize promoter or control sequences normally associated with the desired gene sequence, provide such control sequences are compatible with the host cell systems.

An origin of replication may be provided either by construction of the vector to include an exogenous origin, such as may be derived from SV40 or other viral (e.g. Polyoma, Adeno, VSV, BPV, etc.) source, or may be provided by the host cell chromosomal replication mechanism. If the vector is integrated into the host cell chromosome, the latter is often sufficient.

B.2 Selection of cell lines

In selecting a preferred host cell for transfection by the vectors of the invention, it is appropriate to select the host according to the type of DHFR protein employed. If wild type DHFR protein is employed, it is preferable to select a host cell which is deficient in DHFR, thus permitting the use of the DHFR coding sequence as a marker for successful transfection in selective medium which lacks hypoxanthine, glycine, and thymidine. An appropriate host cell in this case is the Chinese hamster ovary (CHO) cell line deficient in DHFR activity, prepared and propagated as described by Urlaub and Chasin, *Proc. Natl. Acad. Sci.* (USA) 77: 4216 (1980), incorporated hereby by reference.

On the other hand, if DHFR protein with low binding affinity for MTX is used as the controlling sequence, it is not necessary to use DHFR resistant cells. Because the mutant DHFR is resistant to methotrexate, MTX containing media can be used as a means of selection provided that the host cells are themselves methotrexate sensitive. Most eukaryotic cells which are capable of absorbing MTX appear to be methotrexate sensitive. One such useful cell line is a CHO line, CHO-K1 ATCC No. CCL 61.

The example which is set forth hereinbelow describes use of CHO cells as host cells, and expression vectors which include the SV40 origin of replication as a promoter. However, it would be well within the skill of the art to use analogous techniques to construct expression vectors for expression of desired protein sequences in alternative eukaryotic host cell cultures.

B.3 Methods employed

If cells without formidable cell wall barriers are used as host cells, transfection is carried out by the calcium phosphate precipitation method as described by Graham and Van der Eb, *Virology*, 52: 546 (1978). However, other methods for introducing DNA into cells such as by nuclear injection or by protoplast fusion may also be used.

If prokaryotic cells or cells which contain substantial cell wall constructions are used, the preferred method of transfection is calcium treatment using calcium chloride as described by Cohen, F. N. et al *Proc. Natl. Acad. Sci.* (USA), 69: 2110 (1972).

Construction of suitable vectors containing the desired coding and control sequences employ standard ligation techniques. Isolated plasmids or DNA fragments are cleaved, tailored, and religated in the form desired to form the plasmids required.

Cleavage is performed by treating with restriction enzyme (or enzymes) in suitable buffer. In general, about 1 μg plasmid or DNA fragments is used with about 1 unit of enzyme in about 20 μl of buffer solution. (Appropriate buffers and substrate amounts for particular restriction enzymes are specified by the manufacturer.) Incubation times of about 1 hour at 37°C are workable. After incubations, protein is removed by extraction with phenol and chloroform, and the nucleic acid is recovered from the aqueous fraction by precipitation with ethanol.

If blunt ends are required, the preparation is treated for 15 minutes at 15° with 10 units of Polymerase I (Klenow), phenol-chloroform extracted, and ethanol precipitated.

Size separation of the cleaved fragments is performed using 6 percent polyacrylamide gel described by Goeddel, D., et al, *Nucleic Acids Res.*, 8: 4057 (1980) incorporated herein by reference.

For ligation approximately equimolar amounts of the desired components, suitably end tailored to provide correct matching are treated with about 10 units T4 DNA ligase per 0.5 μg DNA. (When cleaved vectors are used as components, it may be useful to prevent religation of the cleaved vector by pretreatment with bacterial alkaline phosphatase.)

The ligation mixture was used to transform *E. coli* K12 strain 294 (ATCC 31446), and successful transformants were selected by ampicillin resistance. Plasmids from the transformants were prepared, analyzed by restriction and/or sequenced by the method of Messing, et al, *Nucleic Acids Res.*, 9:309 (1981) or by the method of Maxam, et al, *Methods in Enzymology*, 65:499 (1980).

Amplification of DHFR protein coding sequences is effected by growing host cell cultures in the presence of approximately 20—500,000 nM concentrations of methotrexate, a competitive inhibitor of DHFR activity. The effective range of concentration is highly dependent, of course, upon the nature of the DHFR gene, protein and the characteristics of the host. Clearly, generally defined upper and lower limits cannot be ascertained. Suitable concentrations of other folic acid analogs or other compounds which inhibit DHFR could also be used. MTX itself is, however, convenient, readily available and effective.

B.4 Results obtainable

The methods of the invention permit the production in host cell cultures of antigenically active tPA protein in amounts greater than 0.1 pg per cell per day. With suitable application of amplifying conditions, amounts greater than 20 pg can be obtained. Stated in alternate terms, gene expression levels resulting in production of more than $9\times10^{-6}$ units, or, with suitable amplification, more than $18\times10^{-5}$ units of tPA activity are achieved.

C. Examples

The following examples are intended to illustrate but not to limit the invention. In the examples here, a CHO cell line suitable for the type of DHFR protein coding sequence to be introduced was employed as a host cell culture in each case. However, other eukaryotic and prokaryotic cells are suitable for the method of the invention as well.

C.1 Production of tPA using DHFR protein with a low binding affinity for MTX
C.1.A Vector construction

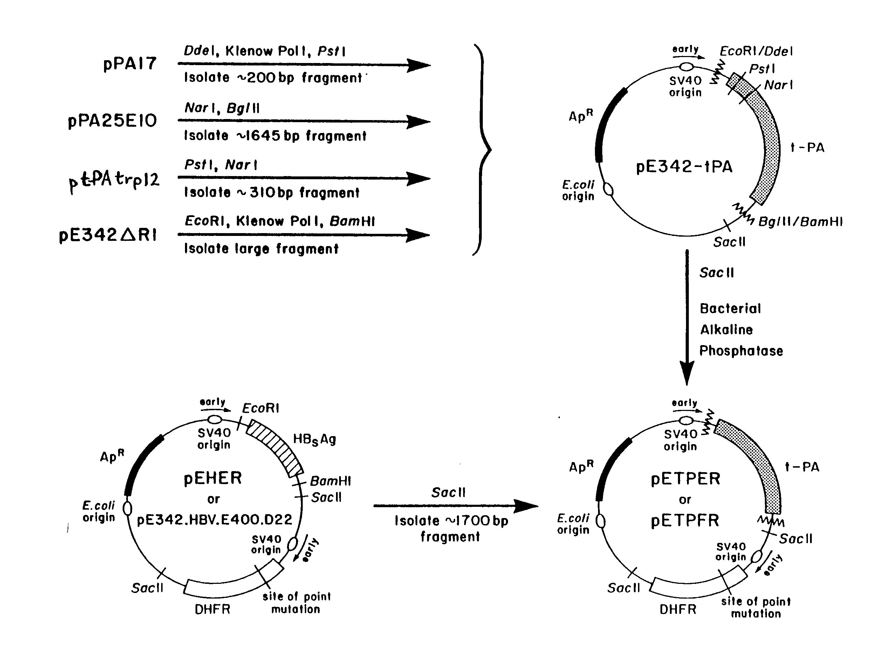

The sequence encoding human tissue plasminogen activator (tPA) is inserted, an expression plasmid for a mutant DHFR with low binding affinity for MTX, described in EP 117060 A, by the following procedure (see Figure 1):

cDNA plasmids encoding tPA have been described by Goeddel et al, European Application Publn. No. 0093619. Human melanoma cells (Bowes)—the melanoma cells were cultured to confluent monolayers in 100 ml Earles Minimal Essential Media supplemented with sodium bicarbonate (0.12 percent final concentration), 2 mM glutamine and 10 percent heat-inactivated fetal calf serum. To confirm that the melanoma cells were actively producing human tissue plasminogen activator, human melanoma cells were cultured to confluency in a 24 well microtiter dish. Either in the presence or absence of 0.33 μM the

protease inhibitor aprotinin, the cells were washed once with phosphate buffered saline and 0.3 ml of serum free methionine free medium was added. 75 µCi of [$^{35}$S]-methionine was added and the cells were labeled at 37° for 3 hours. At the end of the 3 hour labelling period the media was removed from the cells and treated with either tissue plasminogen activator specific IgG or pre-immune serum for immunoprecipitation (54). The immunoprecipitated products were displayed by electrophoresis on a 10 percent SDS-acrylamide gel. The slab gel was fixed, dried and subjected to fluorography.

Total RNA from melanoma cell cultures was extracted essentially as reported by Ward *et al., J. Virol. 9*, 61 (1972). Cells were pelleted by centrifugation and then resuspended in 10 mM NaCl, 10 mM Tris-HCl pH 7.5, 1.5 mM $MgCl_2$. Cells were lysed by the addition of NP-40 (1 percent final concentration), and nuclei were pelleted by centrifugation. The supernatant contained the total RNA which was further purified by multiple phenol and chloroform extractions. The aqueous phase was made 0.2 M in NaCl and then total RNA was precipitated by the addition of two volumes of ethanol. Oligo-dT cellulose chromatography was utilized to purify mRNA from the total RNA preparations Crea *et al., Nucl. Acids Res. 8*, 2331 (1980). Typical yields from 10 grams of cultured melanoma cells were 5 to 10 milligrams of total RNA and 50—200 micrograms of Poly(A) plus mRNA.

Fractionation of PolyA$^+$ mRNA (200 µg) (Aniv *et al., PNAS 69*, 1408 (1972)) was performed by electrophoresis through urea-agarose gels. The slab agarose gel was composed of 1.75 percent agarose, 0.025 M sodium citrate, pH 3.8 and 6 M urea. Electrophoresis was performed for 7 hours at 25 milliamps and 4°C. The gel was then fractionated with a razor blade. The individual slices were melted at 70° and extracted twice with phenol and once with chloroform. Fractions were then ethanol precipitated and subsequently assayed by *in vitro* translation in a rabbit reticulocyte lysate system, Bethesda Research Lab. supplemented with dog pancreas microsomes as follows: Translations were performed using 25 µCi of [$^{35}$S] methionine and 500 nanograms of each gel slice RNA in a final volume of 30 µl containing 25 mM HEPES, 48.3 mM potassium chloride, 10 mM creating phosphate. 19 amino acids at 50 mM each, 1.1 mM magnesium chloride 16.6 mM EDTA 0.16 mM dithiothreitol 8.3 mM hemin, 16.6 µg/ml creatine kinase, 0.33 mM calcium chloride, 0.66 mM EGTA, 23.3 mM sodium chloride.

Incubations were carried out at 30°C for 90 minutes. Dog pancreas microsomal membranes prepared from rough microsomes using EDTA for removal of the ribosomes Blobel *et al., J. Cell Biol. 67*, 852 (75) were treated with nuclease Shields *et al., J. Biol. Chem. 253*, 3753 (78) and were present in the translation mixture at a final concentration of 7 $A_{260}$ units/ml. Translation products or immunoprecipitated translation products were analyzed by electrophoresis on 10 percent polyacrylamide gels in sodium dodecyl sulfate as previously described (Laemmli, *Nature 227*, 680 (70)). The unstained slab gels were fixed, dried and subjected to fluorography (Bonner *et al., Eur. J. Biochem. 46*, 83 (74)).

The resulting translation products from each gel fraction were immunoprecipitated with rabbit antihuman tissue plasminogen activator specific IgG. One major immunoprecipitated polypeptide band was observed in the translation of RNA fraction numbers 7 and 8 (migration of 21 to 24S) having a molecular weight of approximately 63,000 daltons. This band was not observed when preimmune IgG was used for immunoprecipitation which suggested these polypeptides were tissue plasminogen activator specific.

Five µg of gel fractionated mRNA (gel slice 7 mRNA) was used for the preparation of double stranded cDNA by standard procedures (52, 65, 66). The cDNA was size fractionated on a 6 percent polyacrylamide gel. The cDNA greater than 350 base pairs in length (125 ng) was electroeluted. 30 ng of cDNA was extended with deoxy(C) residues using terminal deoxynucleotidyl transferase and annealed with 300 ng of the plasmid pBR322 which had been similarly tailed with deoxy(G) residues at the *Pst* I site (Chang *et al., Nature 275*, 617 (78)). The annealed mixture was then transformed into E. coli K12 strain 294 (ATCC No. 31446). Approximately 4,600 transformants were obtained.

Purified human tissue plasminogen activator was obtained according to the procedure of disclosed references (EPO Publn. 41776).

The molecule was scanned in order to locate regions best suited for making synthetic probes, as follows:

To make the proteins susceptible to digestion by trypsin it was reduced and carboxymethylated. A 2 mg sample of tissue plasminogen activator was first dialyzed against 0.01 percent Tween 80 over night at room temperature. The lyophilized protein was then dissolved in 12 ml of 0.56 M Tris-HCl buffer (pH 8.6), 8 molar in urea and 5 mM EDTA. The disulfide bonds were reduced by the addition of 0.1 ml of β-mercaptoethanol. This reaction was carried out under nitrogen for 2 hours at 45°C. The reduced disulfides were alkylated to the carboxymethyl derivative by the addition of 1.0 ml of 1.4 M iodoacetic acid in 1N NaOH. After 20 min at room temperature the reaction was stopped by dialysis against 0.01 percent Tween 80 for 18 hours at room temperature and lyophilized.

The resulting lyophilized carboxymethylated protein was redissolved in 3 ml of 0.1 M sodium phosphate buffer (pH 7.5). Trypsin (TPCK) was added (1 to 50 ratio) and digested at 37°C. Aliquots (0.1 ml) were taken at 3 hours, 6 hours, and 12 hr. A second addition of trypsin was made at 12 hr. The reaction was stopped after 24 hr by freezing the sample until it could be injected on the HPLC. The progress of the digestion was determined by SDS gels on the aliquots. All gels were blank except for a faint band on the 3 hour aliquot. This indicated that the 24 hour digestion was complete and no large peptides remained.

A sample (ca. 0.5 ml) was injected into a high resolution Altex C-8 ultrasphere 5 µ column with two runs. A gradient of acetonitrile was made gradual (1 percent to 5 percent in 5 min, 5 percent to 35 percent in 100 min, 35—50 percent in 30 min). In one of the two preparative runs, the eluant was monitored at two wavelengths (210 nm and 280 nm). The ratio of the two wavelength absorptions was used to indicate the tryptophan containing peptides.

The peptide peaks most likely to contain tryptophan, or that were believed useful for other reasons, were sequenced first. This enabled the determination of the sequence around most of the tryptophans. After sequencing after 25 of the best possible peptide peaks, all the sequence data that could be aligned was pooled to obtain a preliminary model of the primary structure of tissue plasminogen activator. From this data and model, several possible probes were located.

The colonies were individually inoculated into wells of microtiter plates containing LB+5 µg/ml tetracycline and stored at −20°C after addition of DMSO to 7 percent. Two copies of the colony library were grown up on nitrocellulose filters and the DNA from each colony fixed to the filter by the Grunstein Hogness procedure (PNAS 72, 3961 (75)).

The $^{32}$P-labelled—TC($^A_G$)CA($^A_G$)TA($^C_T$)TCCCA probe was prepared (from the synthetic oligomer) (W-E-Y-C-D) 14-mer pool as described above. Filters containing 4,600 transformants were prehybridized for 2 hours at room temperature in 50 mm sodium phosphate pH 6.8, 5X SSC (Blin et al., Nucl. Acids Res. 3, 2303 (76)), 150 µg/ml sonicated salmon sperm DNA, 5X Denhardt's solution 10 percent formamide and then hybridized with $50X10^6$ counts per minute of the labelled probe in the same solution. After an overnight incubation at room temperature, the filters were washed 3 times at room temperature in 6X SSC, 0.1 percent SDS for 30 minutes, once in 2X SSC and then exposed to Kodak XR-5 x-ray film with Dupont Lightning Plus itensifying screens for 16 hours.

Plasmid DNA was isolated by a rapid method (Brinboim et al., Nucl. Acids Res. 7, 1513 (79)) from all colonies showing a positive hybridization reaction. The cDNA inserts from these clones were then sequenced after subcloning fragments into the M13 vector mp 7 (Messing et al., Nucl. Acids Res. 9, 309 ('81)) and by the Maxam Gilbert chemical procedure. The cDNA insert in clone 25E10 was demonstrated to be the DNA coding for tissue plasminogen activator by comparing its amino acid sequence with peptide sequence (See Supra) obtained from purified tissue plasminogen activator and by its expression product produced in E. coli as described in more detail infra. Clone 25E10 was 2304 basa pairs in length with the longest open reading frame encoding a protein of 508 amino acids (MW of 56,756) and containing a 772 bp 3' untranslated region. This cDNA clone lacked the N-terminal coding sequences.

50 µg of pPA25E10 (supra) were digested with Pst I and the 376 bp fragment isolated by electrophoresis on a 6 percent polyacrylamide gel. Approximately 3 µg of this fragment was isolated from the gel by electroeluting, digested with 30 units of Dde I for 1 hr at 37° phenol and chloroform extracted, and ethanol precipitated. The resulting Dde I sticky ends were extended to blunt ends by adding 5 units of DNA polymerase I (Klenow fragment) and 0.1 mM each of dATP, dCTP, dGTP, dTTP to the reaction mixture and incubating at 4°C for 8 hours. After extraction with phenol and chloroform, the DNA was digested with 15 units of Nar I for 2 hours and the reaction mixture electrophoresed on a 6 percent polyacrylamide gel. Approximately 0.5 µg of the desired 125 bp blunt end Nar I fragment was recovered. This fragment codes for amino acids number 69 through 110 of the mature full length tissue plasminogen activator protein.

For isolation of the 1645 bp Nar I—Bgl II fragment, 30 µg of pPA25E10 were digested with 30 units of Nar I and 35 units of Bgl II for 2 hours at 37° and the reaction mixture electrophoresed on a 6 percent polyacrylamide gel. Approximately 6 µg of the desired 1645 bp Nar I—Bgl II fragment were recovered.

The plasmid pdeltaR1SRC is a derivative of the plasmid pSRCex16 (McGrath et al., Nature 295, 423 (82)) in which the Eco R1 sites proximal to the trp promoter and distal to the SRC gene have been removed by repair with DNA polymerase I, and the self-complementary oligodeoxynucleotide AATTATGAATTCAT (synthesized by the phosphotriester method) was inserted into the remaining Eco RI site immediately adjacent to the Xba I site. 20 µg of pdeltaR1SRC were digested to completion with Eco RI, phenol and chloroform extracted, and ethanol precipitated. The plasmid was then digested with 100 units of nuclease S1 at 16°C for 30 minutes in 25 mM sodium acetate (pH 4.6), 1 mM $ZnCl_2$ and 0.3 M NaCl to create a blunt end with the sequence ATG. After phenol and chloroform extraction and ethanol precipitation, the DNA was digested with Bam H1, electrophoresed on a 6 percent polyacrylamide gel, and the large (4,300 bp) vector fragment recovered by electroelution.

The expression plasmid was assembled by ligating together 0.2 µg of vector, 0.06 µg of the 125 bp blunt end—Nar I fragment and 0.6 µg of the 1645 bp Nar I—Bgl II fragment with 10 units of $T_4$ DNA ligase for 7 hours at room temperature and used to transform E. coli strain 294 (ATCC No. 31446) to ampicillin resistance. Plasmid DNA was prepared from 26 of the colonies and digested with Xba I and Eco RI. Twelve of these plasmids contained the desired 415 bp Xba I—Eco RI and 472 bp Eco RI-fragments. DNA sequence analysis verified that several of these plasmids had an ATG initiation codon correctly placed at the start of amino acid number 69 (serine). One of these plasmids, pΔRIPA° was tested and produced the desired tissue plasminogen activator.

0.4 µg of the synthetic oligonucleotide 5' TTCTGAGCACAGGGCG 3' was used for priming 7.5 µg of gel fraction number 8 mRNA (supra) to prepare double stranded cDNA by standard procedures. The cDNA was size fractionated on a 6 percent polyacrylamide gel. A size fraction greater than 300 base pairs (36 ng) was electroeluted. 5 ng cDNA was extended with deoxy (C) residues using terminal deoxycytidyl transferase and annealed with 50 ng of the plasmid pBR322 which had been similarly tailed with deoxy (G) residues at the Pst I site. The annealed mixture was then transformed into E. coli K12 strain 294. Approximately 1,500

8

transformants were obtained.

Since the cDNA priming reaction had been done using a synthetic fragment that hybridized 13 base pairs from the N-terminal of clone pPA25E10, no convenient restriction fragment was available in this 29 base pair region (which includes the 16-mer sequence) for screening the cDNA clones. Therefore, it was necessary to isolate a human tissue plasminogen activator genomic clone in order to identify any primer extended cDNA clones containing N-terminal tissue plasminogen activator coding sequences.

The first step in this process involved establishing the fact that only a single homologous tissue plasminogen activator gene is present in human genomic DNA. To determine this, a Southern hybridization was performed. In this procedure, 5 μg of high molecular weight human lymphocyte DNA was digested to completion with various restriction endonucleases, electrophoresed on 1.0 percent agarose gels and blotted to a nitrocellulose filter. A $^{32}$P-labelled DNA probe was prepared from the 5' end of the cDNA insert of the cDNA clone pPA25E10 (a 230 bph Hpa II—RSA I fragment) and hybridized (82) with the nitrocellulose filter. $35 \times 10^6$ counts per minute of the probe were hybridized for 40 hours and then washed (Fritsch et al., Cell 19, 959 (80)). Two endonuclease digestion patterns provide only a single hybridizing DNA fragment: Bgl II (5.7 Kbp) and Pvu II (4.2 Kbp). Two hybridizing DNA fragments were observed with Hinc II (5.1 Kbp and 4.3 Kbp). Taken together, these data suggest the presence of only a single tissue plasminogen activator gene in the human genome, and that this gene contains at least one intervening sequence.

The strategy used to identify λ phage recombinants carrying tissue plasminogen activator genes consisted in detecting nucleotide homology with a radioactive probe prepared from the tissue plasminogen activator clone pPA25E10. One million recombinant λ phage were plated out on DP 50 Sup F at a density of 10,000 pfu/15 cm plate, and nitrocellulose filter replicas were prepared for each plate by the method of Benton and Davis (Science 196, 180 (77)). A $^{32}$P-labelled DNA probe was prepared by standard procedures from a 230 base pair Hpa II—Rsa I fragment located 34 base pairs from the 5' end of the clone p25E10. Each nitrocellulose filter was prehybridized at 42° for 2 hours in 50 mM sodium phosphate (pH 6.5), 5X SSC (77), .05 mg/ml sonicated salmon sperm DNA, 5X Denhardt's solution, 50 percent formamide and then hybridized with $50 \times 10^6$ counts per minute of the labelled probe in the same solution containing 10 percent sodium dextran sulfate. After an overnight incubation at 42°C, the filters were washed 4 times at 50° in 0.2X SSC, 0.1 percent SDS for 30 minutes, once in 2×SSC at room temperature and then exposed to Kodak XR-5 X-ray film with Dupont Cronex intensifying screens overnight. A total of 19 clones were obtained which hybridized with the probe. Phage DNA was prepared as previously described from 6 recombinants. λ Clone C was selected for preparation of a Pvu II fragment for colony screening. 30 μg of DNA was digested with Pvu II for 1 hour at 37°, and electrophoresed on 1.0 percent agarose gels. A 4.2 Kilobase pair fragment previously shown to contain tissue plasminogen activator sequences was electroeluted and purified. A $^{32}$P labelled probe was prepared by standard procedures for colony hybridizations as described infra.

The colonies were transferred from plates and grown on nitrocellulose filters and the DNA from each colony fixed to the filter by the Grunstein—Hogness procedure. A $^{32}$P-labelled probe was made by calf-thymus priming Taylor et al., Biochem. Biophy. Acta 442, 324 (76) a 4.2 kilobase pair Pvu II fragment from an isolated tissue plasminogen activator λ genomic clone. Filters containing the 1,500 transformants were hybridized with $112 \times 10^6$ cpm of $^{32}$P-genomic Pvu II fragment. Hybridization was for 16 hours using conditions described by Fritsch et al. Filters were extensively washed and then exposed to Kodak XR-5 X-ray film with Dupont Lightning-Plus intensifying screens for 16—48 hours. Eighteen colonies clearly hybridized with the genomic probe. Plasmid DNA was isolated from each of these colonies and was bound to nitrocellulose filters and hybridized with the $^{32}$P-labelled synthetic oligonucleotide (16-mer) used for the original priming reaction. Of the 18 clones, seven hybridized with the kinased 16-mer. Upon sequence analysis after subcloning fragments into the m13 vector mp[7] (73), one clone (pPA17) was shown to contain the correct 5' N terminal region of tissue plasminogen activator, a signal leader sequence and an 84 bp 5' untranslated region. From the two clones pPA25E10 and pPA17 the complete nucleotide sequence and restriction pattern of a full length tissue plasminogen activator clone were determined, and a plasmid pt-pAtrp 12 containing the complete coding sequence was constructed as described more fully in EP93619.

Three fragments from overlapping tPA plasmids, pPA25E10, pPA17 and pt-PAtrp12 were prepared as follows: Plasmid pPA17 was digested with Dde I, filled in using Klenow DNA polymerase 1, and subcut with Pst I; the approximately 200 bp fragment containing 5' terminal tPA sequence thus generated was isolated. The second tPA fragment was obtained by digesting pt-PAtrp12 with Pst I and Nar I and isolating the approximately 310 bp fragment. The third tPA fragment was obtained by digesting pPA25E10 with Nar I and Bgl II and isolating the approximately 1645 bp fragment which contains, in addition to much of the tPA coding region, some 3' non-translated sequences.

Plasmid p342E which expresses HBV surface antigen (also referred to as pHBs348-E) has been described by Levinson et al, patent application Serial No. 326,980, filed December 3, 1981, which is incorporated herein by reference. pE342 is modified by digesting with trace amounts of Eco RI, filling in the cleaved site using Klenow DNA polymerase I, and ligating the plasmid back together, thus removing the Eco RI site preceding the SV40 origin in pE342. The resulting plasmid, designated pE342ΔR1, is digested with Eco RI, filled in using Klenow DNA polymerase I, and subcut with Bam HI. After electrophoresing on acrylamide gel, the approximately 3500 bp fragment is electroeluted, phenol-chloroformed, and ethanoled as above.

Plasmid pE342 which expresses HBV surface antigen (also referred to as pHBs348-E) has been described by Levinson et al, patent application Serial No. 326,980, filed December 3, 1981, which is incorporated herein by reference (EPO Publn. 0073656). (Briefly, the origin of the Simian virus SV40 was isolated by digesting SV40 DNA with HindIII, and converting the HindIII ends to EcoRI ends by the addition of a converter (AGCTGAATTC). This DNA was cut with PvuII, and RI linkers added. Following digestion with EcoRI, the 348 base-pair fragment spanning the origin was isolated by polyacrylamide gel electrophoresis and electroelution, and cloned in pBR322. Expression plasmid pHBs348-E was constructed by cloning the 1986 base-pair fragment resulting from EcoRI and BglII digestion of HBV (*Animal Virus Genetics*, (Ch. 5) Acad. Press, N.Y. (1980)) (which spans the gene encoding HBsAg) into the plasmid pML (Lusky *et al.*, *Nature*, 293: 79 (1981) at the EcoRI and BamHI sites. (pML is a derivative of pBR322 which has a deletion eliminating sequences which are inhibitory to plasmid replication in monkey cells). The resulting plasmid (pRI-Bgl) was then linearized with EcoRI, and the 348 base-pair fragment representing the SV40 origin region was introduced into the EcoRI site of pRI-Bgl. The origin fragment can insert in either orientation. Since this fragment encodes both the early and late SV40 promoters in addition to the origin of replication, HBV genes could be expressed under the control of either promoter depending on this orientation (pHBS348-E representing HBs expressed under control of the early promoter). pE342 is modified by partially digesting with Eco RI, filling in the cleaved site using Klenow DNA polymerase I, and ligating the plasmid back together, thus removing the Eco RI site preceding the SV40 origin in pE342. The resulting plasmid, designated pE342ΔR1, is digested with Eco RI, filled in using Klenow DNA polymerase I, and subcut with Bam HI. After electrophoresing on acrylamide gel, the approximately 3500 bp fragment is electroeluted, phenol-chloroform extracted, and ethanol precipitated as above.

The thus prepared p342E 3500 bp vector, and above described tPA fragments comprising approximately 2160 bp were ligated together using standard techniques. A plasmid containing the three tPA encoding fragments in the proper orientation was isolated, characterized, and designated pE342-t-PA. This plasmid was digested with Sac II and treated with bacterial alkaline phosphatase (BRL). To provide the DHFR sequence (along with control sequences for its expression) an approximately 1700 bp fragment was generated by SacII digestion of pEHER. (pEHER is a plasmid expressing mutant DHFR described in EP 117060A.)

In the construction of pEHER, p342E is described in Levinson *et al.* EPO Publication No. 0073656, March 9, 1983; pHBV-T-1A and pSVR are described by Liu *et al.*, *DNA*, 1: 213 (1982); and pFR400 is prepared as follows.

The 540 bp Hind II—Hind III fragment encompassing the SV40 origin of replication (Liu *et al.* DNA 1:213 (1982)) was ligated into plasmid pML (Lusky, M. and Botchan, M., *Nature* 293: 79 (1981)) between the EcoR I site and the Hind III site. The plasmid Eco RI site and SV40 Hind II site were made blunt by the addition of Klenow DNA polymerase I in the presence of the 4 dNTPs prior to digestion with Hind III. The resulting plasmid, pESV was digested with Hind III and Bam HI and the 2900 bp vector fragment isolated. To this fragment was ligated a Hind III—Bgl II fragment of 2025 bp form HBV modified to contain a polylinker (DNA fragment containing multiple restriction sites) at the Eco RI site. The HBV fragment encompasses the surface antigen gene and is derived by Eco RI-Bgl II digestion of cloned HBV DNA described by Liu *et al.*, *supra* (DNA-1: 213, 1982). The doubled stranded linker DNA fragment (5'dAAGCTTATCGATTCTAGAATTC3'...) was digested with Hind III and Eco RI and added to the HBV fragment, converting the Eco RI—Bgl II fragment to a Hind III—Bgl II fragment. Although this could be done as a 3 part ligation consisting of linker, HBV fragment, and vector, it is more convenient and was so performed to first add the Hind III—Eco RI linker to the cloned HBV DNA and then excise the Hind III—Bgl II fragment by codigestion of the plasmid with those enzymes. The resulting plasmid, pCVESVHBV, contains a bacterial origin of replication from the pBR322 derived pML, and ampicillin resistance marker, also from pML, an SV40 fragment oriented such that the early promoter will direct the transcription of the ingested HBV fragment, and the surface antigen gene from HBV. The HBV fragment also provides a polyadenylation signal for the production of polyadenylated mRNAs such as are normally formed in the cytoplasm of mammalian cells. The HBsAg coding region is removed by digesting with Eco RI, filling-in the ends with Klenow DNA polymerase as previously described, and subcutting with the BamHI. Into this area is inserted the Fnu 4HI-Bgl II fragment from DHFR encoding cDNA. The resulting plasmids are diagrammed in Figure 14. pFD11 was constructed using the Fnu4HI-Bgl II fragment of wildtype DHFR cDNA plasmid pDHFR-11 (Nunberg, *et al.*, (Cell 19: 355 (1980)). pFR400 was constructed using the similar fragment from pR400.12.

pR400.12 is a recombinant plasmid containing a DNA sequence encoding a methotrexate resistant DHFR. It is prepared by isolating mRNA from mutant 3T6R400 cells (Haber, D. A. and Schimke, R. T., *Cell*, 26: 355 (1981)), preparing a cDNA library from the isolated mRNA, ligating the cDNA into Pst cleaved pBR322, transforming *E. coli* strain 294 (ATCC 31446) and probing the transformants with the Pst I-Bgl II digest of the cDNA insert from murine DHFR cDNA (Nunberg, J. H. *et al.* (supra)), and selecting the colony which contains plasmids with the correct mutant DHFR coding sequence."

This fragment was ligated into the pE342-t-PA plasmid to create pEdPAER400, a plasmid which is analogous to pEHER except that the HBsAg coding region has been replaced by the cDNA sequences from t-PA.

pETPAER400 (pETpER) was transfected into both dhfr⁻ CHO-DUX B11 cells and DHFR+ CHO-K1 (ATCC CCL61) cells. Transformed dhfr⁻ cells were selected by growth in glycine, hypoxanthine and thymidine deficient medium. Transformed DHFR+ cells were selected by growth in ≥100 nM MTX. Colonies which arose on the appropriate selection medium were isolated using cloning rings and propagated in the same medium to several generations.

For amplification cells from the colonies are split into media containing $5 \times 10^4$, $10^5$, $2.5 \times 10^5$, $5 \times 10^5$, and $10^6$ nM MTX and passaged several times. Cells are plated at very low ($10^2$—$10^3$ cells/plate) cell densities in 10 cm dishes and the resulting colonies are isolated.

In a manner analogous to that used in the construction of pETPER, a plasmid containing the DNA sequence encoding wild type DHFR, pETPER, was constructed. The construction was as described in Example C.1.A except that in place of plasmid pEHER as a source for the DHFR protein gene sequence, the plasmid pE342.HBV.E400.D22. The plasmid pE342.HBV.E400.D22 is the same as pEHER except for a single base pair difference between wild type and mutant DHFR. It can be constructed analogously to pEHER, using pFD11 in place of pFR400. Thus the resulting plasmid pETPFR is analogous in every way to pETPER except that the DNA sequence encoding for wild type DHFR is substituted for that of the mutant.

### C.1.B Expression and amplification of the tPA sequence

pETPAER400 (pETPER) was transfected into both dhfr⁻ (CHO-DUX B11) obtained by permission from Urlaub and Chasin, and DHFR+ CHO-K1 (ATCC CCL61) cells by the method of Graham and Van der Eb (supra). Transformed dhfr⁻ cells were selected by growth in glycine, hypoxanthine and thymidine deficient medium. Transformed DHFR+ cells were selected by growth in ≥100 nM MTX. Colonies which arose on the appropriate selection medium were isolated using cloning rings and propagated in the same medium to several generations.

For amplification cells from the colonies are split into media containing $5 \times 10^4$, $10^5$, $2.5 \times 10^5$, $5 \times 10^5$, and $10^6$ nM MTX and passaged several times. Cells are plated at very low ($10^2$—$10^3$ cells/plate) cell densities in 10 cm dishes and the resulting colonies are isolated as usual.

### C.1.C Assay methods

Expression of tPA in the transfected amplified colonies may conveniently be assayed by the methods set forth in U.S. Application 398003 (EP 0093619). Briefly, for quantitative assay, the medium or extract to be tested is placed in a solution containing plasminogen, and the amount of plasmin formed is measured by monitoring the cleavage of a chromogenic substrate such as S2251, Kabi Group Inc., Greenwich, CT. An aliquot of the sample is mixed with 0.1 ml of 0.7 mg/ml plasminogen (in 0.5M Tris-HCl, pH 7.4, containing 0.012M NaCl) and the volume adjusted to 0.15 ml. The mixture is incubated at 37°C for ten minutes, 0.35 ml of S2251 (1.0 mM solution in the above buffer) is added and the reaction continued for 30 minutes at 37°C. Acetic acid (25 µl) is added to terminate the reaction. The samples are centrifuged and the absorbance at 405 nm is measured. Quantitation of the amount of activity is obtained by comparison with a standard urokinase solution. The assay conditions for detection of a full length plasminogen activator were modified by the addition of fibrinogen (0.2 mg) to the solution. Fibrinogen results in a stimulation of the activity of plasminogen activator observed, therefore resulting in somewhat elevated levels of activity. Activity was recorded in Plough units, wherein 90,000 Plough units is equal to the activity exhibited by 1 mg of purified tissue plasminogen activator.

Coamplification of DHFR and tPA sequences is assayed by isolating DNA from confluent monolayers of amplified colonies as follows: Confluent monolayers in 150 mm plates are washed with 50 ml sterile PBS and lysed by the addition of 5 ml of 0.1 percent SDS, 0.4 M CaCl₂, 0.1 M EDTA, pH 8. After 5—10 minutes, the mixture is removed, phenol extracted, chloroform extracted, and ethanol precipitated. The DNA is resuspended in 1 ml (per 150 mm plate) 10 mM Tris pH 8, 1 mM EDTA (TE), RNase added to 0.1 mg/ml, and the solution incubated 30 minutes at 37°. SDS is then added to 0.1 percent and pronase (Sigma) is added to 0.5 mg/ml. After 3—16 hours incubation at 37°, the solution is again phenol extracted, chloroform extracted, and ethanol precipitated as usual. The DNA pellet is resuspended in 0.5 ml water and digested with restriction enzymes as per the standard protocol. Approximately 5—10 µg of digested DNA is electrophoresed in an agarose gel [1 percent agarose in Tris—acetate buffer (40 mM Tris, 1 mM EDTA, made to pH 8.2 with acetic acid)]; Crouse, et al, *J. Biol. Chem.,* 257: 7887 (1982)). After bromphenol blue dye had migrated 2/3 of the way down the gel, the gel is removed and stained with ethidium bromide. After visualizing the DNA was ultraviolet light, the gel is treated with HCl, NaOH, and NaCl-Tris and transferred to nitrocellulose filters according to the procedure of Southern (*J. Mol Biol.* 98: 503. (1975)). The filters are then hybridized with a nick translated probe made from the 1700 bp SacII fragment of pEHER (prepared and hybridized as described above), or from the approximately 1970 bp Bgl II fragment of pETPER.

### C.2 Production of tPA in conjunction with wild type DHFR protein
### C.2.A Vector construction

In a manner exactly analogous to that used in the construction of pETPER, a plasmid containing the DNA sequence encoding wild type DHFR, pETPFR, was constructed. The construction was exactly as described in Example C.1.A except that in place of plasmid pEHER as a source for the DHFR protein gene sequence, the plasmid pE342.HBV.E400.D22 described in EP 117058A was substituted. The plasmid pE342.HBV.E400.D22 is exactly the same as pEHER except for a single base pair difference between wild type and mutant DHFR. Thus the resulting plasmid pETPFR is analogous in every way to pETPER except that the DNA sequence encoding for wild type DHFR is substituted for that of the mutant.

C.2.B Expression of tPA sequence

pETPFR was used to transfect DHFR deficient CHO cells (Urlaub and Chasin (supra)) using the calcium phosphate precipitation method of Graham and Van der Eb. Twenty-one colonies which arose on the selective medium (-HGT) were assayed by detection of plasmin formation as assessed by the digestion of fibrin in an agar plate containing fibrin and plasminogen, described by Granelli-Piperno, et al., *J. Exp. Med.,* 148: 223 (1978).

Four of the best positive clones were then assayed quantitatively for plasmin formation on a per cell basis according to the method set forth in C.1.C.

Upon such quantitative determination it was found that the four clones tested exhibited the same or comparable tPA secretion into the medium, determined as units/cell/day. Subclones were prepared by transferring inocula from two of the clones into separate plates containing -HGT medium. Two of the resulting subclones, 18B and 1 were used for further analysis.

C.2.C Amplification and tPA production levels

The above subclones were plated at $2 \times 10^5$ cells per 100 mm plates in 50 nM MTX to promote amplification. Those cells which survived, when assayed as described above, gave, in all cases, about 10 times the unamplified amount of plasminogen activator activity. Two of these clones were chosen for further study and were named 1—15 and 18B-9.

Subclone 1—15 was further amplified by seeding $2 \times 10^5$ cells in 100 mm plates containing 500 nM MTX. Assay of the cells thus amplified yielded a further increase (of about 3 fold) in tPA production; when assayed quantitatively by the method of C.1.C, levels were in the range of $7 \times 10^{-4}$ units/cell/day. A portion of these amplified cells was then transferred and maintained in the presence of 10,000 nM MTX. Subclones of 1—15, and 18B-9 were further tested after being maintained for approximately 1—2 months at the conditions specified in Table 1.

## TABLE 1

| Cell line | Growth conditions | ng tPA/cell/day* |
|---|---|---|
| 1—15$_{500}$ | 500 nM MTX | $28.5 \times 10^{-3}$ |
| 1—15$_{500}$ | 500 nM MTX | $26.0 \times 10^{-3}$ |
| 1—15$_{500}$ | (-HGT medium, no MTX) | $8.3 \times 10^{-3}$ |
| 1—15$_{500}$ | (-HGT medium, no MTX) | $18.0 \times 10^{-3}$ |
| 1—15$_{10,000}$ | 10 µM MTX | $29.3 \times 10^{-3}$ |
| 1—15$_{10,000}$ | 10 µM MTX | $49.0 \times 10^{-3}$ |
| 18B-9 | 50 nM MTX | $14.3 \times 10^{-3}$ |
| 18B-9 | 50 nM MTX | $14.4 \times 10^{-3}$ |
| 18B-9 | (-HGT medium, no MTX) | $14.3 \times 10^{-3}$ |
| 18B-9 | (-HGT medium, no MTX) | $14.4 \times 10^{-3}$ |
| 1 | (-HGT medium, no MTX) | $1.0 \times 10^{-3}$ |
| 1 | (-HGT medium, no MTX) | $0.7 \times 10^{-3}$ |

*tPA in the culture medium was assayed quantitatively in a radioimmunoassay as follows: Purified tPA and purified iodinated tracer tPA derived from melanoma cells were diluted serially to include concentration of 12.5 to 400 ng/ml in a buffer containing phosphate buffered saline, pH 7.3, 0.5 percent bovine serum albumin, 0.01 percent Tween 80, and 0.02 percent NaN3. Appropriate dilutions of medium samples to be assayed were added to the radioactively labelled tracer proteins. The antigens were allowed to incubate overnight at room temperature in the presence of a 1:10,000 dilution of the IgG fraction of a rabbit anti-tPA antiserum. Antibody-antigen complex was precipitated by absorption to goat anti-rabbit IgG Immunobeads (BioRad) for two hours at room temperature. The beads were cleared by the addition of saline diluent followed by centrifugation for ten minutes at 2000×g at 4° Celsius. Supernatants were discarded and the radioactivity in the precipitates was monitired. Concentrations were assigned by comparison with the reference standard.

The cell lines are as follows: Cell line "1" is an unamplified clone from the original set of four. "1—15$_{500}$" is an amplified subclone of cell line "1" which was amplified initially in 50 nM MTX to give 1—15 and then transferred for further amplification into 500 nM MTX. 1—15$_{10,000}$ is subclone of 1—15$_{500}$ which has been further amplified in the presence of 10,000 nM MTX. Cell line 18B-9 is a subclone of one of the original four detected which had been amplified on 50 nM MTX.

All of the amplified cells show increased levels of TPA production over that exhibited by the unamplified cell culture. Even the unamplified culture produces amounts of tPA greater than 0.5 pg/cell/day; amplification results in levels approaching 50 pg/cell/day.

**Claims for the Contracting States: BE, CH, DE, FR, GB, IT, LI, LU, NL, SE**

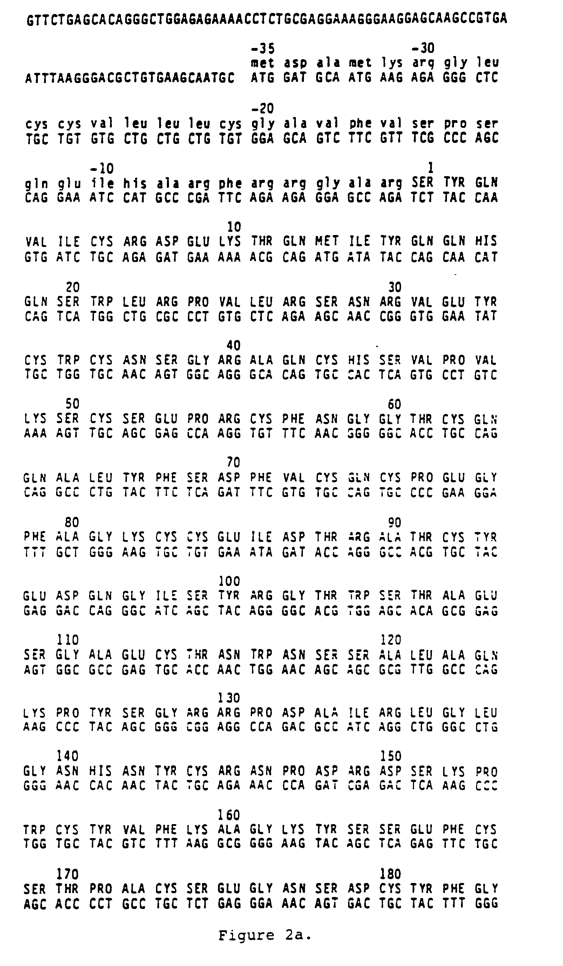

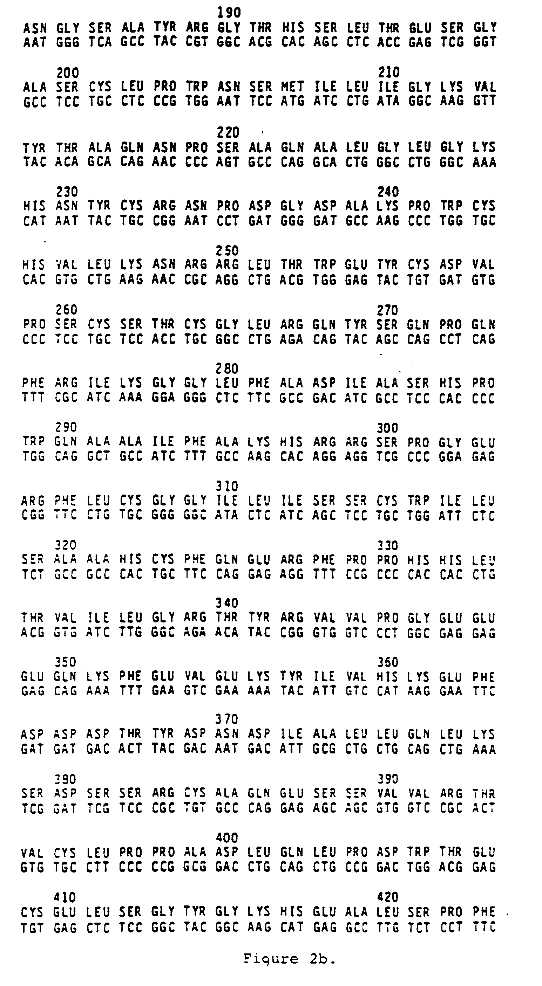

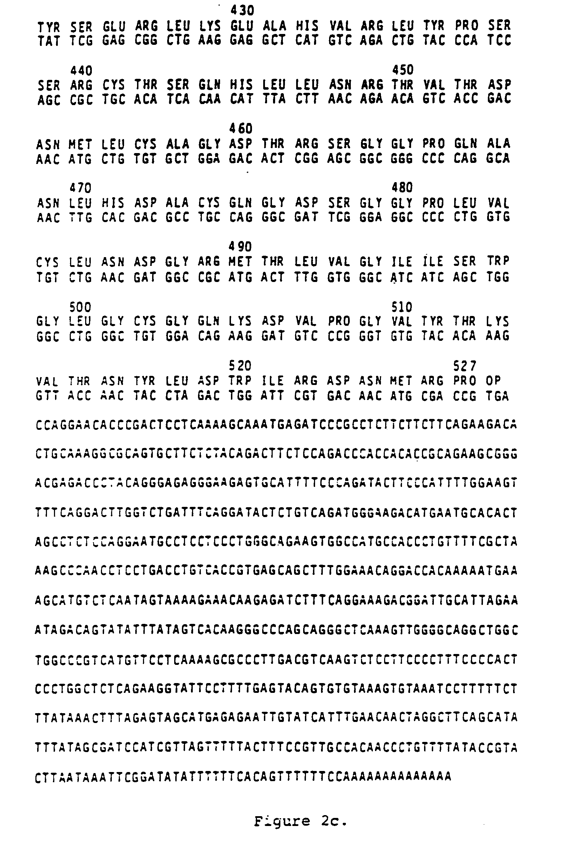

1. An expression vector which comprises:
(i) a first DNA sequence encoding a DHFR protein; and
(ii) a second DNA sequence encoding human t-PA of the amino acid sequence depicted in Figure 2 hereof or a derivative thereof by way of amino acid deletion substitution, insertion, inversion, addition or replacement and having human tissue plasminogen activator function; wherein each of said first and second sequences is operably linked to a DNA sequence capable of effecting its expression.

2. The vector of claim 1 wherein the DHFR protein is wild-type DHFR.

3. The vector of claim 2 which is effective in cells deficient in DHFR.

4. The vector of claim 1 wherein the DHFR protein has a low binding affinity for MTX.

5. The vector of claim 4 which is effective in cells sensitive to MTX.

6. A plasmid as shown diagrammatically for pETPER in Figure 1 hereof, wherein:
$A_p^R$ is the *E. coli* ampicillin resistance gene;
"early" indicates the SV40 early promoter, oriented in the direction indicated by the arrow;
DHFR is the coding sequence for a mutant DHFR gene which differs from the wild-type gene by a base pair mutation which imparts increased resistance to methotrexate, said coding sequence being oriented so as to be under the control of the adjacent SV40 early promoter;
t-PA is the entire coding sequence for human tissue plasminogen activator as defined in claim 1; said coding sequence being oriented so as to be under the control of the adjacent SV40 early promoter; the plasmid having both SV40 and *E. coli* origins of replication, so as to be replicable in both *E. coli* and mammalian host cells.

7. A plasmid as shown diagrammatically for pETPFR in Figure 1 hereof, wherein:
$A_p^R$ is the *E. coli* ampicillin resistance gene;
"early" indicates the SV40 early promoter, oriented in the direction indicated by the arrow;
DHFR is the coding sequence for the wild-type DHFR gene, said coding sequence being oriented so as to be under the control of the adjacent SV40 early promoter;
t-PA is the entire coding sequence for human tissue plasminogen activator as defined in claim 1; said coding sequence being oriented so as to be under the control of the adjacent SV40 early promoter;
the plasmid having both SV40 and *E. coli* origins of replication, so as to be replicable in both *E. coli* and mammalian host cells.

8. Recombinant cells as obtainable by transfection with a vector of any one of claims 1 to 7 so as to be capable of expressing both DNA sequences.

9. A method of preparing t-PA which comprises growing recombinant cells according to claim 8 under conditions suitable for said expression, and recovering t-PA from said cells.

10. A method of amplifying a DNA sequence encoding t-PA in recombinant cells transfected with a vector of any one of claims 1 to 7, which method comprises growing the cells in the presence of an effective amplifying concentration of MTX.

11. A method of increasing the production of t-PA in a recombinant cell culture of claim 8, which method comprises growing the cells in the presence of an effective amplifying concentration of MTX.

**Claims for the Contracting State: AT**

1. A process which comprises the preparation of an expression vector which comprises:
(i) a first DNA sequence encoding a DHFR protein; and
(ii) a second DNA sequence encoding human t-PA of the amino acid sequence depicted in Figure 2 hereof or a derivative thereof by way of amino acid deletion substitution, insertion, inversion, addition or replacement und having human tissue plasminogen activator function; wherein each of said first and second sequences is operably linked to a DNA sequence capable of effecting its expression.

2. The process of claim 1 wherein the DHFR protein is wild-type DHFR.

3. The process of claim 2 wherein the vector is effective in cells deficient in DHFR.

4. The process of claim 1 wherein the DHFR protein has a low binding affinity for MTX.

5. The process of claim 4 wherein the vector is effective in cells sensitive to MTX.

6. A process which comprises the preparation of a plasmid as shown diagrammatically for pETPER in Figure 1 hereof, wherein:
$A_p^R$ is the *E. coli* ampicillin resistance gene;

"early" indicates the SV40 early promoter, oriented in the direction indicated by the arrow;

DHFR is the coding sequence for a mutant DHFR gene which differs from the wild-type gene by a base pair mutation which imparts increased resistance to methotrexate, said coding sequence being oriented so as to be under the control of the adjacent SV40 early promoter;

t-PA is the entire coding sequence for human tissue plasminogen activator as defined in claim 1; said coding sequence being oriented so as to be under the control of the adjacent SV40 early promoter;

the plasmid having both SV40 and *E. coli* origins of replication, so as to be replicable in both *E. coli* and mammalian host cells.

7. A process which comprises the preparation of a plasmid as shown diagrammatically for pETPFR in Figure 1 hereof, wherein:

A_p^R is the *E. coli* ampicillin resistance gene;

"early" indicates the SV40 early promoter, oriented in the direction indicated by the arrow;

DHFR is the coding sequence for the wild-type DHFR gene, said coding sequence being oriented so as to be under the control of the adjacent SV40 early promoter;

t-PA is the entire coding sequence for human tissue plasminogen activator as defined in claim 1; said coding sequence being oriented so as to be under the control of the adjacent SV40 early promoter;

the plasmid having both SV40 and *E. coli* origins of replication, so as to be replicable in both *E. coli* and mammalian host cells.

8. Recombinant cells as obtained by transfection with a vector as obtainable by the process of any one of claims 1 to 7 so as to be capable of expressing both DNA sequences.

9. A method of preparing t-PA which comprises growing recombinant cells according to claim 8 under conditions suitable for said expression, and recovering t-PA from said cells.

10. A method of amplifying a DNA sequence encoding t-PA in recombinant cells transfected with a vector as obtainable by the process of any one of claims 1 to 7, which method comprises growing the cells in the presence of an effective amplifying concentration of MTX.

11. A method of increasing the production of t-PA in a recombinant cell culture of claim 8, which method comprises growing the cells in the presence of an effective amplifying concentration of MTX.

**Patentansprüche für die Vertragsstaaten: BE, CH, DE, FR, GB, IT, LI, LU, NL, SE**

1. Verfahren zur Herstellung eines Expressionsvektors, der umfaßt:

(1) eine erste DHA Sequenz, die ein DHFR-Protein kodiert; und

(ii) eine zweite DNA-Sequenz, die Human-tPA der in Fig. 2 der vorliegenden Beschreibung dargestellten Aminosäuresequenz oder ein Derivat derselben durch Aminosäurelöschung, -substitution, -einfügung, -inversion, -zugabe oder -austausch und mit einer Human-Gewebeplasminogenaktivator-Funktion kodiert; worin jede der genannten ersten und zweiten Sequenzen operabel an eine DNA-Sequenz gebunden ist, die fähig ist, ihre Expression zu bewirken.

2. Verfahren nach Anspruch 1, worin das DHFR-Protein DHFR des wilden Typs ist.

3. Verfahren nach Anspruch 2, worin der Vektor wirksam in Zellen ist, die einen Mangel an DHFR aufweisen.

4. Verfahren nach Anspruch 1, worin das DHFR-Protein eine geringe Bindungsauffinität für MTX besitzt.

5. Verfahren nach Anspruch 4, worin der Vektor wirksam in Zellen ist, die empfindlich gegenüber MTX sind.

6. Verfahren zur Herstellung eines Plasmids, das in Fig. 1 der vorliegenden Beschreibung schematisch für pETPER dargestellt ist, worin

A_p^R das E. coli Ampicillinresistenzgen ist;

"early" den SV40 Frühpromotor bedeutet, der in der durch den Pfeil angegebenen Richtung orientiert ist;

DHFR die Kodierungssequenz für ein mutantes DHFR-Gen ist, das sich vom Gen des wilden Typs durch eine Basenpaarmutation unterscheidet, die erhöhte Resistenz gegenüber Methotrexat verleiht, wobei die genannte Kodierungssequenz so orientiert ist, daß sie unter der Steuerung des benachbarten SV40 Frühpromotors steht;

t-PA die gesamte Kodierungssequenz für Human-Gewebeplasminogenaktivator wie in Anspruch 1 definiert ist; wobei die genante Kodierungssequenz so ausgerichtet ist, daß sie unter der Steuerung des benachbarten SV40 Frühpromotors steht;

und das Plasmid sowohl SV40 als auch E. coli Replikationsursprünge aufweist, sodaß es sowohl in E. coli als auch in Säugetierwirtszellen replizierbar ist.

7. Verfahren zur Herstellung eines Plasmids, das in Fig. 1 der vorliegenden Beschreibung schematisch für pETPFR dargestellt ist, worin

A_p^R das E. coli Ampicillinresistenzgen ist;

"early" den SV40 Frühpromotor bedeutet, der in der durch den Pfeil angegebenen Richtung orientiert ist;

DHFR die Kodierungssequenz für das DHFR-Gen des wilden Typs ist, wobei die genannte

14

Kodierungssequenz so orientiert ist, daß sie unter der Steuerung des benachbarten SV40 Frühpromotors steht;

t-PA die gesamte Kodierungssequenz für Human-Gewebeplasminogenaktivator wie in Anspruch 1 definiert ist; wobei die genannte Kodierungssequenz so orientiert ist, daß sie unter der Steuerung des benachbarten SV40 Frühpromotors steht;

und das Plasmid sowohl SV40 als auch E. coli Replikationsursprünge besitzt, sodaß es sowohl in E. coli als auch in Säugetierwirtszellen replizierbar ist.

8. Rekominante Zellen, die durch Transfektion mit einem Vektor nach einem der Ansprüche 1 bis 7 erhältlich sind, um fähig zu sein, beide DNA-Sequenzen zu exprimieren.

9. Verfahren zur Herstellung von t-PA umfassend das Kultivieren von rekombinanten Zellen nach Anspruch 8 unter Bedingungen, die für die genannte Expression geeignet sind, und Gewinnung des t-PA aus den genannten Zellen.

10. Verfahren zur Verstärkung einer DNA-Sequenz, die t-PA in rekombinanten Zellen kodiert, die mit einem Vektor nach einem der Ansprüche 1 bis 7 transfiziert sind, welches Verfahren das Kultivieren der Zellen in Gegenwart einer wirksamen verstärkenden Konzentration von MTX umfaßt.

11. Verfahren zur Steigerung der Produktion von t-PA in einer rekombinanten Zellenkultur nach Anspruch 8, welches Verfahren das Kultivieren der Zellen in Gegenwart einer wirksamen verstärkenden Konzentration von MTX umfaßt.

**Patentansprüche für den Vertragsstaat: AT**

1. Verfahren zur Herstellung eines Expressionsvektors, der umfaßt:
(1) eine erste DNA Sequenz, die ein DHFR-Protein kodiert; und
(ii) eine zweite DNA-Sequenz, die Human-tPA der in Fig. 2 der vorliegenden Beschreibung dargestellten Aminosäuresequenze oder ein Derivat derselben durch Aminosäurelöschung, -substitution, -einfügung, -inversion, -zugabe oder -austausch und mit einer Human-Gewebeplasminogenaktivator-Funktion kodiert; worin jede der genannten ersten und zweiten Sequenzen operabel an eine DNA-Sequenz gebunden ist, die fähig ist, ihre Expression zu bewirken.

2. Verfahren nach Anspruch 1, worin das DHFR-Protein DHFR des wilden Typs ist.

3. Verfahren nach Anspruch 2, worin der Vektor wirksam in Zellen ist, die einen Mangel an DHFR aufweisen.

4. Verfahren nach Anspruch 1, worin das DHFR-Protein eine geringe Bindungsaffinität für MTX besitzt.

5. Verfahren nach Anspruch 4, worin der Vektor wirksam in Zellen ist, die empfindlich gegenüber MTX sind.

6. Verfahren zur Herstellung eines Plasmids, das in Fig. 1 der vorliegenden Beschreibung schematisch für pETPFR dargestellt ist, worin

$A_p{}^R$ das E. coli Ampicillinresistenzgen ist;

"early" den SV40 Frühpromotor bedeutet, der in der durch den Pfeil angegebenen Richtung orientiert ist;

DHFR die Kodierungssequenz für ein mutantes DHFR-Gen ist, das sich vom Gen des wilden Typs durch eine Gasenpaarmutation unterscheidet, die erhöhte Resistenz gegenüber Methotrexat verleiht, wobei die genannte Kodierungssequenz so orientiert ist, daß sie unter der Steuerung des benachbarten SV40 Frühpromotors steht;

t-PA die gesamte Kodierungssequenz für Human-Gewebeplasminogenaktivator wie in Anspruch 1 definiert ist; wobei die genante Kodierungssequenz so ausgerichtet ist, daß sie unter der Steuerung des benachbarten SV40 Frühpromotors steht;

und das Plasmid sowohl SV40 als auch E. coli Replikationsursprünge aufweist, sodaß es sowohl in E. coli als auch in Säugetierwirtszellen replizierbar ist.

7. Verfahren zur Herstellung eines Plasmids, das in Fig. 1 der vorliegenden Beschreibung schematisch für pETPFR dargestellt ist, worin

$A_p{}^R$ das E. coli Ampicillinresistenzgen ist;

"early" den SV40 Frühpromotor bedeutet, der in der durch den Pfeil angegebenen Richtung orientiert ist;

DHFR die Kodierungssequenz für das DHFR-Gen des wilden Typs ist, wobei die genannte Kodierungssequenz so orientiert ist, daß sie unter der Steuerung des benachbarten SV40 Frühpromotors steht;

t-PA die gesamte Kodierungssequenz für Human-Gewebeplasminogenaktivator wie in Anspruch 1 definiert ist; wobei die genannte Kodierungssequenz so orientiert ist, daß sie unter der Steuerung des benachbarten SV40 Frühpromotors steht;

und das Plasmid sowohl SV40 als auch E. coli Replikationsursprünge besitzt, sodaß es sowohl in E. coli als auch in Säugetierwirtszellen replizierbar ist.

8. Rekombinante Zellen, die durch Transfektion mit einem Vektor nach einem der Ansprüche 1 bis 7 erhältlich sind, um fähig zu sein, beide DNA-Sequenzen zu exprimieren.

9. Verfahren zur Herstellung von t-PA umfassend das Kultivieren von rekombinanten Zellen nach Anspruch 8 unter Bedingungen, die für die genannte Expression geeignet sind, und Gewinnung des t-PA aus den genannten Zellen.

10. Verfahren zur Verstärkung einer DNA-Sequenz, die t-PA in rekombinanten Zellen kodiert, die mit einem Vektor nach einem der Ansprüche 1 bis 7 transfiziert sind, welches Verfahren das Kultivieren des Zellen in Gegenwart einer wirksamen verstärkenden Konzentration von MTX umfaßt.

11. Verfahren zur Steigerung der Produktion von t-PA in einer rekombinanten Zellenkultur nach Anspruch 8, welches Verfahren das Kultivieren der Zellen in Gegenwart einer wirksamen verstärkenden Konzentration von MTX umfaßt.

**Revendications pour les États Contractants: BE, CH, DE, FR, GB, IT, LI, LU, NL, SE**

1. Vecteur d'expression qui comprend:

(i) une première séquence d'ADN qui code une protéine DHFR, et

(ii) une deuxième séquence d'ADN qui code un tPA humain de la séquence d'acides aminés représentée sur la figure 2 annexée ou un dérivé de celle-ci, par suppression, substitution, insertion, inversion, addition ou remplacement d'un acide aminé, et qui a une fonction d'activateur du plasminogène du tissu humain,

caractérisé en ce que chacune desdites première et deuxième séquences est liée de manière fonctionnelle à une séquence d'ADN capable d'effectuer son expression.

2. Vecteur selon la revendication 1, caractérisé en ce que la protéine DHFR est une DHFR de type sauvage.

3. Vecteur selon la revendication 2, caractérisé en ce qu'il est efficace dans des cellules présentant un manque de DHFR.

4. Vecteur selon la revendication 1, caractérisé en ce que la protéine DHFR a une faible affinité de liaison avec le MTX.

5. Vecteur selon la revendication 4, caractérisé en ce qu'il est efficace dans des cellules sensibles au MTX.

6. Plasmide, tel qu'il est représenté schématiquement pour pETPER sur la figure 1 annexée, dans lequel:

- $A_p^R$ représente le gène de résistance à l'ampicilline de *E. coli*;

"précoce" indique l'activateur précoce SV40, orienté dans la direction indiquée par la flèche;

DHFR est la séquence de codage pour un gène DHFR mutant qui diffère du gène de type sauvage par une mutation d'une paire de bases qui transmet une résistance accrue au méthotrexate, ladite séquence de codage étant orientée de façon à être sous le contrôle de l'activateur précoce adjacent SV40;

tPA est la séquence de codage complète de l'activateur du plasminogène du tissu humain tel qu'il est défini dans la revendication 1, ladite séquence de codage étant orientée de façon à être sous le contrôle de l'activateur précoce adjacent SV40;

le plasmide ayant à la fois des origines de réplication de SV40 et de *E. coli*, de sorte qu'il est apte à une réplication à la fois dans des cellules de *E. coli* et dans des cellules-hôtes de mammifères.

7. Plasmide, tel qu'il est représenté schématiquement pour pETPFR sur la figure 1 annexée, dans lequel:

$A_p^R$ représente le gène de résistance à l'ampicilline de *E. coli*;

"précoce" indique l'activateur précoce SV40, orienté dans la direction indiquée par la flèche;

DHFR est la séquence de codage pour le gène DHFR de type sauvage, ladite séquence de codage étant orientée de façon à être sous le contrôle de l'activateur précoce adjacent SV40;

tPA est la séquence de codage complète de l'activateur du plasminogène du tissu humain tel qu'il est défini dans la revendication 1, ladite séquence de codage étant orientée de façon à être sous le contrôle de l'activateur précoce adjacent SV40;

le plasmide ayant à la fois des origines de réplication de SV40 et de *E. coli*, de sorte qu'il est apte à une réplication à la fois dans des cellules de *E. coli* et dans des cellules-hôtes de mammifères.

8. Cellules de recombinaison pouvant être obtenues par transfection au moyen d'un vecteur selon l'une quelconque des revendications 1 à 7 de manière à pouvoir exprimer les deux séquences d'ADN.

9. Méthode de préparation de tPA, consistant à cultiver des cellules de recombinaison selon la revendication 8, dans des conditions appropriées à ladite expression, et à recueillir le tPA desdites cellules.

10. Méthode d'amplification d'une séquence d'ADN codant le tPA dans des cellules de recombinaison obtenues par transfection au moyen d'un vecteur selon l'une quelconque des revendications 1 à 7, caractérisée en ce qu'elle comprend la mise en culture des cellules en présence d'une concentration de MTX d'amplification effective.

11. Méthode d'accroissement de la production de tPA dans une culture de cellules de recombinaison selon la revendication 8, caractérisée en ce qu'elle comprend la mise en culture des cellules en présence d'une concentration de MTX d'amplification effective.

**Revendications pour l'État Contractant: AT**

1. Procédé comprenant la préparation d'un vecteur d'expression qui comprend:

(i) une première séquence d'ADN qui code une protéine DHFR, et

16

(ii) une deuxième séquence d'ADN qui code un tPA humaine de la séquence d'acides aminés représentée sur la figure 2 annexée ou un dérivé de celle-ci, par suppression, substitution, insertion, inversion, addition ou remplacement d'un acide aminé, et qui a une fonction d'activateur du plasminogène du tissu humain,

caractérisé en ce que chacune desdites première et deuxième séquences est liée de manière fonctionnelle à une séquence d'ADN capable d'effectuer son expression.

2. Procédé selon la revendication 1, caractérisé en ce que la protéine DHFR est une DHFR de type sauvage.

3. Procédé selon la revendication 2, caractérisé en ce que le vecteur est efficace dans des cellules présentant un manque de DHFR.

4. Procédé selon la revendication 1, caractérisé en ce que la protéine DHFR a une faible affinité de liaison avec le MTX.

5. Procédé selon la revendication 4, caractérisé en ce que le vecteur est efficace dans des cellules sensibles au MTX.

6. Procédé comprenant la préparation d'un plasmide, tel qu'il est représenté schématiquement pour pETPER sur la figure 1 annexée, dans lequel:

$A_p^R$ représente le gène de résistance à l'ampicilline de *E. coli*;

"précoce" indique l'activateur précoce SV40, orienté dans la direction indiquée par la flèche;

DHFR est la séquence de codage pour un gène DHFR mutant qui diffère du gène de type sauvage par une mutation d'une paire de bases qui transmet une résistance accrue au méthotrexate, ladite séquence de codage étant orientée de façon à être sous le contrôle de l'activateur précoce adjacent SV40;

tPA est la séquence de codage complète de l'activateur du plasminogène du tissu humain tel qu'il est défini dans la revendication 1, ladite séquence de codage étant orientée de façon à être sous le contrôle de l'activateur précoce adjacent SV40;

le plasmide ayant à la fois des origines de réplication de SV40 et de *E. coli*, de sorte qu'il est apte à une réplication à la fois dans des cellules de *E. coli* et dans des cellules-hôtes de mammifères.

7. Procédé comprenant la préparation d'un plasmide, tel qu'il est représenté schématiquement pour pETPFR sur la figure 1 annexée, dans lequel:

$A_p^R$ représente le gène de résistance à l'ampicilline de *E. coli*;

"précoce" indique l'activateur précoce SV40, orienté dans la direction indiquée par la flèche;

DHFR est la séquence de codage pour le gène DHFR de type sauvage, ladite séquence de codage étant orientée de façon à être sous la contrôle de l'activateur précoce adjacent SV40;

tPA est la séquence de codage complète de l'activateur du plasminogène du tissu humain tel qu'il est défini dans la revendication 1, ladite séquence de codage étant orientée de façon à être sous le contrôle de l'activateur précoce adjacent SV40;

le plasmide ayant à la fois des origines de réplication de SV40 et de *E. coli*, de sorte qu'il est apte à une réplication à la fois dans des cellules de *E. coli* et dans des cellules-hôtes de mammifères.

8. Cellules de recombinaison pouvant être obtenues par transfection au moyen d'un vecteur selon l'une quelconque des revendications 1 à 7 de manière à pourvoir exprimer les deux séquences d'ADN.

9. Méthode de préparation de tPA, consistant à cultiver des cellules de recombinaison selon la revendication 8, dans des conditions appropriées à ladite expression, et à recueillir le tPA desdites cellules.

10. Méthode d'amplification d'une séquence d'ADN codant le tPA dans des cellules de recombinaison obtenues par transfection au moyen d'un vecteur selon l'une quelconque des revendications 1 à 7, caractérisée en ce qu'elle comprend la mise en culture des cellules en présence d'une concentration de MTX d'amplification effective.

11. Méthode d'accroissement de la production de tPA dans une culture de cellules de recombinaison selon la revendication 8, caractérisée en ce qu'elle comprend la mise en culture des cellules en présence d'une concentration de MTX d'amplification effective.

pPAI7 —— *DdeI, Klenow PolI, PstI* ——→
Isolate ∼200 bp fragment

pPA25EIO —— *NarI, BglII* ——→
Isolate ∼1645 bp fragment

p t-PA trpI2 —— *PstI, NarI* ——→
Isolate ∼310 bp fragment

pE342△RI —— *EcoRI, Klenow PolI, BamHI* ——→
Isolate large fragment

early *EcoRI/DdeI*
SV40 origin *PstI*
*NarI*
Ap^R
pE342-tPA t-PA
E.coli origin
*BglII/BamHI*
*SacII*

*SacII*

Bacterial
Alkaline
Phosphatase

early *EcoRI*
SV40 origin HB_S Ag
Ap^R
pEHER
or *BamHI*
pE342.HBV.E400.D22 *SacII*
E.coli origin
SV40 origin early
*SacII*
DHFR site of point mutation

—— *SacII* ——→
Isolate ∼1700 bp fragment

early
SV40 origin t-PA
Ap^R
pETPER
or
pETPFR
E.coli origin
*SacII*
SV40 origin early
*SacII*
DHFR site of point mutation

```
GTTCTGAGCACAGGGCTGGAGAGAAAACCTCTGCGAGGAAAGGGAAGGAGCAAGCCGTGA


                                        -35                    -30
                                        met asp ala met lys arg gly leu
         ATTTAAGGGACGCTGTGAAGCAATGC     ATG GAT GCA ATG AAG AGA GGG CTC


                                        -20
         cys cys val leu leu leu cys gly ala val phe val ser pro ser
         TGC TGT GTG CTG CTG CTG TGT GGA GCA GTC TTC GTT TCG CCC AGC

                 -10                                              1
         gln glu ile his ala arg phe arg arg gly ala arg SER TYR GLN
         CAG GAA ATC CAT GCC CGA TTC AGA AGA GGA GCC AGA TCT TAC CAA


                                        10
         VAL ILE CYS ARG ASP GLU LYS THR GLN MET ILE TYR GLN GLN HIS
         GTG ATC TGC AGA GAT GAA AAA ACG CAG ATG ATA TAC CAG CAA CAT


             20                                      30
         GLN SER TRP LEU ARG PRO VAL LEU ARG SER ASN ARG VAL GLU TYR
         CAG TCA TGG CTG CGC CCT GTG CTC AGA AGC AAC CGG GTG GAA TAT


                                        40
         CYS TRP CYS ASN SER GLY ARG ALA GLN CYS HIS SER VAL PRO VAL
         TGC TGG TGC AAC AGT GGC AGG GCA CAG TGC CAC TCA GTG CCT GTC


             50                                      60
         LYS SER CYS SER GLU PRO ARG CYS PHE ASN GLY GLY THR CYS GLN
         AAA AGT TGC AGC GAG CCA AGG TGT TTC AAC GGG GGC ACC TGC CAG


                                        70
         GLN ALA LEU TYR PHE SER ASP PHE VAL CYS GLN CYS PRO GLU GLY
         CAG GCC CTG TAC TTC TCA GAT TTC GTG TGC CAG TGC CCC GAA GGA


             80                                      90
         PHE ALA GLY LYS CYS CYS GLU ILE ASP THR ARG ALA THR CYS TYR
         TTT GCT GGG AAG TGC TGT GAA ATA GAT ACC AGG GCC ACG TGC TAC


                                        100
         GLU ASP GLN GLY ILE SER TYR ARG GLY THR TRP SER THR ALA GLU
         GAG GAC CAG GGC ATC AGC TAC AGG GGC ACG TGG AGC ACA GCG GAG


             110                                     120
         SER GLY ALA GLU CYS THR ASN TRP ASN SER SER ALA LEU ALA GLN
         AGT GGC GCC GAG TGC ACC AAC TGG AAC AGC AGC GCG TTG GCC CAG


                                        130
         LYS PRO TYR SER GLY ARG ARG PRO ASP ALA ILE ARG LEU GLY LEU
         AAG CCC TAC AGC GGG CGG AGG CCA GAC GCC ATC AGG CTG GGC CTG


             140                                     150
         GLY ASN HIS ASN TYR CYS ARG ASN PRO ASP ARG ASP SER LYS PRO
         GGG AAC CAC AAC TAC TGC AGA AAC CCA GAT CGA GAC TCA AAG CCC


                                        160
         TRP CYS TYR VAL PHE LYS ALA GLY LYS TYR SER SER GLU PHE CYS
         TGG TGC TAC GTC TTT AAG GCG GGG AAG TAC AGC TCA GAG TTC TGC


             170                                     180
         SER THR PRO ALA CYS SER GLU GLY ASN SER ASP CYS TYR PHE GLY
         AGC ACC CCT GCC TGC TCT GAG GGA AAC AGT GAC TGC TAC TTT GGG
```

Figure 2a.

```
                                    190
ASN GLY SER ALA TYR ARG GLY THR HIS SER LEU THR GLU SER GLY
AAT GGG TCA GCC TAC CGT GGC ACG CAC AGC CTC ACC GAG TCG GGT


    200                                             210
ALA SER CYS LEU PRO TRP ASN SER MET ILE LEU ILE GLY LYS VAL
GCC TCC TGC CTC CCG TGG AAT TCC ATG ATC CTG ATA GGC AAG GTT


                                    220
TYR THR ALA GLN ASN PRO SER ALA GLN ALA LEU GLY LEU GLY LYS
TAC ACA GCA CAG AAC CCC AGT GCC CAG GCA CTG GGC CTG GGC AAA


    230                                             240
HIS ASN TYR CYS ARG ASN PRO ASP GLY ASP ALA LYS PRO TRP CYS
CAT AAT TAC TGC CGG AAT CCT GAT GGG GAT GCC AAG CCC TGG TGC


                                    250
HIS VAL LEU LYS ASN ARG ARG LEU THR TRP GLU TYR CYS ASP VAL
CAC GTG CTG AAG AAC CGC AGG CTG ACG TGG GAG TAC TGT GAT GTG


    260                                             270
PRO SER CYS SER THR CYS GLY LEU ARG GLN TYR SER GLN PRO GLN
CCC TCC TGC TCC ACC TGC GGC CTG AGA CAG TAC AGC CAG CCT CAG


                                    280
PHE ARG ILE LYS GLY GLY LEU PHE ALA ASP ILE ALA SER HIS PRO
TTT CGC ATC AAA GGA GGG CTC TTC GCC GAC ATC GCC TCC CAC CCC


    290                                             300
TRP GLN ALA ALA ILE PHE ALA LYS HIS ARG ARG SER PRO GLY GLU
TGG CAG GCT GCC ATC TTT GCC AAG CAC AGG AGG TCG CCC GGA GAG


                                    310
ARG PHE LEU CYS GLY GLY ILE LEU ILE SER SER CYS TRP ILE LEU
CGG TTC CTG TGC GGG GGC ATA CTC ATC AGC TCC TGC TGG ATT CTC


    320                                             330
SER ALA ALA HIS CYS PHE GLN GLU ARG PHE PRO PRO HIS HIS LEU
TCT GCC GCC CAC TGC TTC CAG GAG AGG TTT CCG CCC CAC CAC CTG


                                    340
THR VAL ILE LEU GLY ARG THR TYR ARG VAL VAL PRO GLY GLU GLU
ACG GTG ATC TTG GGC AGA ACA TAC CGG GTG GTC CCT GGC GAG GAG


    350                                             360
GLU GLN LYS PHE GLU VAL GLU LYS TYR ILE VAL HIS LYS GLU PHE
GAG CAG AAA TTT GAA GTC GAA AAA TAC ATT GTC CAT AAG GAA TTC


                                    370
ASP ASP ASP THR TYR ASP ASN ASP ILE ALA LEU LEU GLN LEU LYS
GAT GAT GAC ACT TAC GAC AAT GAC ATT GCG CTG CTG CAG CTG AAA


    380                                             390
SER ASP SER SER ARG CYS ALA GLN GLU SER SER VAL VAL ARG THR
TCG GAT TCG TCC CGC TGT GCC CAG GAG AGC AGC GTG GTC CGC ACT


                                    400
VAL CYS LEU PRO PRO ALA ASP LEU GLN LEU PRO ASP TRP THR GLU
GTG TGC CTT CCC CCG GCG GAC CTG CAG CTG CCG GAC TGG ACG GAG


    410                                             420
CYS GLU LEU SER GLY TYR GLY LYS HIS GLU ALA LEU SER PRO PHE
TGT GAG CTC TCC GGC TAC GGC AAG CAT GAG GCC TTG TCT CCT TTC
```

Figure 2b.

```
                                430
TYR SER GLU ARG LEU LYS GLU ALA HIS VAL ARG LEU TYR PRO SER
TAT TCG GAG CGG CTG AAG GAG GCT CAT GTC AGA CTG TAC CCA TCC

     440                                        450
SER ARG CYS THR SER GLN HIS LEU LEU ASN ARG THR VAL THR ASP
AGC CGC TGC ACA TCA CAA CAT TTA CTT AAC AGA ACA GTC ACC GAC

                                460
ASN MET LEU CYS ALA GLY ASP THR ARG SER GLY GLY PRO GLN ALA
AAC ATG CTG TGT GCT GGA GAC ACT CGG AGC GGC GGG CCC CAG GCA

     470                                        480
ASN LEU HIS ASP ALA CYS GLN GLY ASP SER GLY GLY PRO LEU VAL
AAC TTG CAC GAC GCC TGC CAG GGC GAT TCG GGA GGC CCC CTG GTG

                                490
CYS LEU ASN ASP GLY ARG MET THR LEU VAL GLY ILE ILE SER TRP
TGT CTG AAC GAT GGC CGC ATG ACT TTG GTG GGC ATC ATC AGC TGG

     500                                        510
GLY LEU GLY CYS GLY GLN LYS ASP VAL PRO GLY VAL TYR THR LYS
GGC CTG GGC TGT GGA CAG AAG GAT GTC CCG GGT GTG TAC ACA AAG

                                520                     527
VAL THR ASN TYR LEU ASP TRP ILE ARG ASP ASN MET ARG PRO OP
GTT ACC AAC TAC CTA GAC TGG ATT CGT GAC AAC ATG CGA CCG TGA

CCAGGAACACCCGACTCCTCAAAAGCAAATGAGATCCCGCCTCTTCTTCTTCAGAAGACA

CTGCAAAGGCGCAGTGCTTCTCTACAGACTTCTCCAGACCCACCACACCGCAGAAGCGGG

ACGAGACCCTACAGGGAGAGGGAAGAGTGCATTTTCCCAGATACTTCCCATTTTGGAAGT

TTTCAGGACTTGGTCTGATTTCAGGATACTCTGTCAGATGGGAAGACATGAATGCACACT

AGCCTCTCCAGGAATGCCTCCTCCCTGGGCAGAAGTGGCCATGCCACCCTGTTTTCGCTA

AAGCCCAACCTCCTGACCTGTCACCGTGAGCAGCTTTGGAAACAGGACCACAAAAATGAA

AGCATGTCTCAATAGTAAAAGAAACAAGAGATCTTTCAGGAAAGACGGATTGCATTAGAA

ATAGACAGTATATTTATAGTCACAAGGGCCCAGCAGGGCTCAAAGTTGGGGCAGGCTGGC

TGGCCCGTCATGTTCCTCAAAAGCGCCCTTGACGTCAAGTCTCCTTCCCCTTTCCCCACT

CCCTGGCTCTCAGAAGGTATTCCTTTTGAGTACAGTGTGTAAAGTGTAAATCCTTTTTCT

TTATAAACTTTAGAGTAGCATGAGAGAATTGTATCATTTGAACAACTAGGCTTCAGCATA

TTTATAGCGATCCATCGTTAGTTTTTACTTTCCGTTGCCACAACCCTGTTTTATACCGTA

CTTAATAAATTCGGATATATTTTTTCACAGTTTTTTTCCAAAAAAAAAAAAAAAA
```

Figure 2c.